# EUROPÄISCHE PATENTSCHRIFT

(11) **EP 1 719 497 B1**
(45) Veröffentlichungstag und Bekanntmachung des Hinweises auf die Patenterteilung: **09.01.2013**
(21) Anmeldenummer: 06007641.1
(22) Anmeldetag: 12.04.2006
(51) Int. Cl.: A61K 6/083, A61K 6/09, A61K 6/00

(54) **Kompositmaterialien mit geringer Schrumpfkraft**
Composite material with low polymerisation shrinkage stress
Matériau composite à force de retrait faible

(30) Priorität: 04.05.2005 DE 102005021332
(43) Veröffentlichungstag der Anmeldung: 08.11.2006
(73) Patentinhaber: Heraeus Kulzer GmbH, 63450 Hanau (DE)
(72) Erfinder: Ruppert, Klaus, Dr., 63477 Maintal (DE); Grundler, Andreas, Dr., 42117 Wuppertal (DE); Reischl, Kurt, 35799 Merenberg (DE); Eck, Michael, 61389 Schmitten (DE); Hohmann, Alfred, 61389 Schmitten (DE); Diefenbach, Christine, 65599 Dornburg (DE)
(74) Vertreter: Kühn, Hans-Christian

(56) Entgegenhaltungen:
- EP-A- 0 486 775
- WO-A-00/21488
- ERNST, C-P, MEYER G R, KLÖCKER K, WILLERSHAUSEN B: "Determination of polymerization shrinkage stress by means of a photoelastic investigation" DENTAL MATERIALS, Bd. 20, 2004, Seiten 313-321, XP002395854

## Beschreibung

Die Erfindung betrifft Kompositmaterialien mit geringer Schrumpfkraft.

Lichthärtende Werkstoffe auf Acrylat-/Methacrylatbasis erfahren bei radikalischer Polymerisation aufgrund des sich bei der Polymerisation reduzierenden Molekülabstandes und der damit einhergehenden Dichteerhöhung einen Volumenschrumpf. Dieser kann durch Zugabe von anorganischen Füllstoffen wie z.B. Dentalgläsern oder pyrogenen Kieselsäuren deutlich reduziert werden, da sich ein reduzierter Monomeranteil pro Volumeneinheit ergibt und die Füllstoffe während der Polymerisation nicht schrumpfen.

Bei Dentalanwendungen ist der Volumenschrumpf von großer klinischer Bedeutung, da durch die Materialschrumpfung Zugkräfte auf die Kavitätenwand übertragen werden. Bei Überschreitung einer Maximalkraft kann diese Schrumpfkraft im Extremfall zur Ablösung von der Kavitätenwand führen. In den dadurch entstandenen Randspalt können Bakterien eindringen und in der Folge Sekundärkaries entstehen.

Betrachtet man den zeitlichen Verlauf der Schrumpfkraft, zeigt sich folgender typischer Befund:

Direkt nach der Polymerisation ergibt sich durch die Volumenschrumpfung ein Initialwert für die Schrumpfkraft, der dann durch Nachpolymerisation innerhalb von ca. 24 h auf einen Maximalwert ansteigt. Anschließend kommt es durch Wasseraufnahme (im Labor bei einer Lagerung in Wasser bzw. im Mund aus dem Speichel) nach einigen Tagen bis Wochen zu einer leichten Volumenexpansion des Komposits; hierdurch können die Spannungskräfte wieder relaxieren und werden auf ein niedrigeres Niveau zurückgeführt.

Hieraus ergibt sich, daß die entscheidende Einflußgröße der maximale Schrumpfspannungswert nach ca. 24 h ist, da dieser die maximale Kraftbelastung des Verbundsystems Komposit/Adhäsiv/Zahn darstellt.

Es hat nicht an Versuchen gefehlt, schrumpfungsarme Dentalmaterialien zur Verfügung zu stellen: DE 199 05 093 A1 empfiehlt den Einsatz via ringöffnende Metathesepolymerisation (ROMP) härtender, bicyclischer Monomere. Laut DE 198 51 038 A1 ist die Schrumpfung durch Zugabe von Acryloylmorpholin, Cumaronharz, Vinylstearat, Polyvinylacetat oder Alkoholtensiden vor der Polymerisation zu bekämpfen. Gemäß US 5,750,590 schrumpfen kationisch polymerisierbare "Oxetane" (Trimethylenoxide) nur in geringem Maß und eignen sich daher ebenfalls für schrumpfreduzierte Dentalmaterialien. US 6,855,197 B2 beschreibt schrumpfreduzierte Füllungswerkstoffe auf Epoxidharzbasis, die nanoskalige anorganische Oxide als Füllstoffe enthalten. Gemäß US 6,709,271 B2 führt die Verwendung einer Füllstoffmischung mit kugelförmigem Füller der Teilchengröße 200-500 nm und Submikron-Füller der Teilchengröße 20-80 nm zu Schrumpf von bis 1,8% nach der Polymerisation.

Der Gegenstand der vorliegenden Anmeldung bezieht sich in erster Linie auf die Schrumpfkraft und deren Reduktion: Neben den oben beispielhaft erörterten werkstofflichen Eigenschaften beeinflussen auch Verarbeitungsparameter die Schrumpfkraft:

### Lichtleistung

In DE 199 13 890 A1 wurde ein Lichthärtegerät mit Pulsbetrieb zur Behebung von Schrumpfungskraftproblemen vorgeschlagen.

Polymerisationskinetik: Bei identischem Kompositmaterial können geringere Schrumpfkräfte durch eine anfänglich langsamere Polymerisation bei geringerer Lichtleistung und späterer Anhebung der Lichtleistung auf den Maximalwert erzielt werden (Soft-Start Polymerisation). Durch die geringere Lichtleistung zu Beginn bleibt das Komposit-Material länger fließfähig und kann damit Spannungen besser kompensieren und abbauen (J. Esthet. Restor. Dent. (2003) 15, 93 - 104). In US 20050065227 A1 wird vermutet, daß bei Verwendung mulitfunktioneller Photoinitiatoren die frühen Stufen des Schrumpfs stattfinden, solange das Material noch elastisch ist. Das soll schließlich zu geringeren Schrumpfspannungen führen.

Geometrie der Versorgung: Schrumpfkräfte können durch die Anwendung einer Inkrementaltechnik beim Aufbau der Versorgung minimiert werden (US 6,783,810 B2). Je mehr Schichten allerdings einzeln gehärtet werden müssen, desto mehr Zeit benötigt der behandelnde Zahnarzt.

Ziel der vorliegenden Erfindung ist die Bereitstellung eines Kompositmaterials für Dentalanwendungen, welches aufgrund seiner werkstofflichen Eigenschaften die Gefahr der Ablösung der Versorgung von der Kavitätenwand durch Verminderung der Maximalkraft der Schrumpfspannung deutlich reduziert.

Erfindungsgemäß wird diese Aufgabe durch folgende Maßnahmen gelöst:
➢ Durch nicht-agglomerierte Nanofüller (z.B. SiO₂, ZrO₂, TiO₂, Al₂O₃) mit Partikelgrößen < 50 nm als Füllstoffkomponente kann im Vergleich zu herkömmlichen Produkten wie z.B. Aerosilen ein deutlich höherer Gesamtfüllstoffgehalt (> 80 Gew. % bis zu 95 Gew.%) erreicht werden, damit sinkt der Anteil an schrumpffähiger Monomermatrix.
➢ Durch ein Füllstoffgemisch aus grob- und feinteiligen Dentalgläsern, welche ein Größenverhältnis von 1:4 bis 1:30, vorzugsweise 1:4 bis 1:20, besonders ca. 1:5 bis 1:10 aufweisen, lässt sich eine bessere Packung der Füllstoffpartikel und damit ein höherer Füllstoffanteil erzielen. Der höhere Füllstoffanteil ergibt einen geringeren Anteil an schrumpfungsfähiger Monomermatrix (s.o.). Der Anteil an feinteiligen Dentalgläsern darf dabei max. 40 % an der Füllstoffmischung betragen.
➢ In Dentalanwendungen wird üblicherweise eine Monomermischung bestehend aus Bis-GMA und TEDMA verwendet. Bis-GMA wird in einem Anteil von 60 - 80 % und TEDMA in einem Anteil von 20 - 40 % verwendet. Hierbei stellt das Bis-GMA die niedrigschrumpfende Hauptkomponente dar, welche allerdings aufgrund der sehr hohen Viskosität mit einem hochschrumpfenden Verdünner (TEDMA) versetzt werden muss. Durch die überwiegende Substitution des hochschrumpfenden Verdünners TEDMA durch UD-MA (Urethandimethacrylat), welches deutlich unreaktiver ist, wird der Volumenschrumpf reduziert. Überraschenderweise steigt trotz der verminderten Reaktivität des UDMA und der als Folge anzunehmenden verminderten Einbindung in das Polymernetzwerk die Löslichkeit nicht an.
➢ Durch die Verwendung von Tricydodecan-Derivaten wie z.B. SR 833S (Sartomer), Plex 6759-O (Röhm) CD-di-HEMA (Bis(methacryloyloxymethyl)tricyclo[5.2.1.0^{2,6}]decan) oder TCD-di-HEA (Bis(acryloyloxymethyl)tricyclo[5.2.1.0^{2,6}]decan) 2-Propenoicacid, (octahydro-4,7-methane-1H-indene-5-diyl)bis(methyleneiminocarbonyloxy-2,1-ethanediyl)ester oder das analoge HEMA-Derivat (TCD-di-HEMA)als Hauptkomponente(n) anstelle von Bis-GMA lassen sich ebenfalls die erfindungsgemäßen schrumpf- und schrumpfkraftreduzierten Dentalmaterialien herstellen.
➢ Zusätzlich zu diesen Maßnahmen kann optional noch der Gehalt an Photoinitiatoren reduziert werden z.B. auf 0,3 oder 0,1 Gew. %. Dadurch verringert sich der Anteil an umgesetzten Monomeren weiter und damit auch der Polymerisationsschrumpf.

Die Erfindung betrifft demgemäß

Kompositmaterialien mit einem Gesamtfüllstoffgehalt von 80 bis 95 Gew.% enthaltend
A) in der Füllstoffkomponente 0,5 bis 10 Gew % nicht-agglomerierte Nanofüller mit Partikelgrö-βen von 1 bis 50 nm;
B) in der Füllstoffkomponente mindestens 60 Gew. % eines Füllstoffgemischs aus 50 bis 90 % grob- und 10 bis 50 % feinteiligen Dentalgläsern, welche ein Größenverhältnis, bezogen auf die mittlere Partikelgröße (d₅₀-Wert), von
   feinteilig zu grobteilig = 1:4 bis 1:30
   aufweisen;
C) als Monomerkomponente eine Monomermischung aus
   i. 60 - 80 % Bis-GMA oder TCD-di-HEMA oder TCD-di-HEA
   ii. 10 bis 18 % UDMA
   iii. Rest TEDMA und/oder multifunktionelle Vernetzer
D) bis 1 % Initiator(en) und
E) optional in der Füllstoffkomponente mindestens ein weiteres Dentalglas mit sich von den grob- und feinteiligen Dentalgläsern unterscheidender Partikelgröße.

Nicht agglomerierte Nanofüller sind an sich bekannt und z.B. in WO 0130305 A1 oder am Beispiel von SiO₂ in DE 196 17 931 A1 beschrieben. Sie gehören erfindungsgemäß vorzugsweise der Gruppe bestehend aus: SiO₂, ZrO₂, TiO₂, Al₂O₃ sowie Mischungen aus mindestens zwei dieser Stoffe an.

Sie können - wie in DE 196 17 931 A1 beschrieben - in organischen Lösungsmitteln dispergiert sein, aber auch in Wasser oder Wasser enthaltenden Lösungsmittelmischungen.

Als Dentalgläser eignen sich besonders Bariumglaspulver und/oder Strontiumglaspulver. Die mittlere Partikelgröße der grobteiligen Dentalgläser beträt vorzugsweise 5-10 µm, insbesondere um 7 µm und die der feinteiligen 0,5 bis 2, insbesondere 1 µm. Optional vorhandene weitere Dentalgläser haben z.B. mittlere Korngrößen von 2-5 oder 10-50 µm.

Die Füllstoffkomponente kann demnach Dentalgläser mit insgesamt drei oder mehr Kornfraktionen aufweisen. Sie kann auch weitere, herkömmliche, auf dem Dentalgebiet übliche Füllstoffe enthalten, wie etwa Quarz-, Glaskeramik- oder Mischungen davon. Darüber hinaus können die Komposite Füllstoffe zur Erzielung einer erhöhten Röntgenopazität enthalten. Die mittlere Partikelgröße des röntgenopaken Füllstoffs liegt vorzugsweise im Bereich von 100 bis 300 nm, insbesondere 180 bis 300 nm. Als röntgenopake Füllstoffe eignen sich z. B. die in der DE 35 02 594 A1 beschriebenen Fluoride der Seltenen Erdmetalle, d. h. die Trifluoride der Elemente 57 bis 71. Ein besonders bevorzugt verwendeter Füllstoff ist Ytterbiumfluorid, insbesondere Ytterbiumtrifluorid mit einer mittleren Partikelgröße von etwa 300 nm. Die Menge des röntgenopaken Füllstoffs beträgt vorzugsweise 10 bis 50 Gew.-%, besonders bevorzugt 20 bis 30 Gew.-%, bezogen auf den Gesamtfüllstoffgehalt.

Außerdem können gefällte Mischoxide, wie beispielsweise ZrO₂/SiO₂, als Füllstoffe eingesetzt werden. Bevorzugt sind Mischoxide mit einer Partikelgröße von 200 bis 300 nm und insbesondere etwa 200 nm. Die Mischoxidpartikel sind vorzugsweise kugelförmig und weisen eine einheitliche Größe auf. Die Mischoxide haben vorzugsweise einen Brechungsindex von 1,52 bis 1,55. Gefällte Mischoxide werden vorzugsweise in Mengen 25 bis 75 Gew.-% und besonders 40 bis 75 Gew.-% verwendet.

Die Füllstoffe sind zur Verbesserung der Haftung zwischen Füllstoff und organischer Matrix bevorzugt silanisiert. Als Haftvermittler eignet sich besonders alpha - Methacryloxypropyltrimethoxysilan. Die Menge des eingesetzten Haftvermittlers richtet sich nach der Art und BET-Oberfläche des Füllstoffs.

Als multifunktionelle Vernetzer kommen außer TEDMA und UDMA in Frage: Diethylenglycol-di(meth)acrylat, Decandioldi(meth)acrylat, Trimethylolpropantri(meth)acrylat, Pentaerythrit-tetra(meth)acrylat, sowie Butandioldi(meth)acrylat, 1,10-Decandioldi(meth)acrylat, 1,12-Dodecandioldi(meth)acrylat.

Zur Initiierung der Polymerisation enthalten die Komposite einen Polymerisationsinitiator, beispielsweise einen Initiator für die radikalische Polymerisation. Je nach Art des verwendeten Initiators können die Mischungen kalt, durch Licht oder heiß polymerisierbar sein.

Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch alpha, alpha '-Azo-bis(isobutyroethylester), Benzpinakol und 2,2'-Dimethylbenzpinakol sind geeignet.

Als Photoinitatoren kommen z.B. Benzoinalkylether oder -ester, Benzilmonoketale, Acylphosphinoxide oder aliphatische und aromatische 1.2-Diketoverbindungen, wie beispielsweise 2,2-Diethoxyacetophenon 9,10-Phenanthrenchinon, Diacetyl, Furil, Anisil, 4,4'-Dichlorbenzil und 4,4'-Dialkoxybenzil oder Campherchinon, in Frage. Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester, oder organische Phosphite.Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Heißhärtung eingesetzt werden.

Als Initiatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet.

Es können auch dual härtende Systeme verwendet werden, z. B. Photoinitiatoren mit Aminen und Peroxiden.

Die Initiatoren werden vorzugsweise in Mengen von 0,01 bis 1 Gew.-% bezogen auf die Gesamtmasse der Mischung verwendet.

Bei der Kaltpolymerisation kann es zweckmäßig sein, wenn das Kompositmaterial aufgeteilt in zwei Komponenten vorliegt, die zur Aushärtung durch Vermischen vorgesehen sind. Es ist auch möglich, das Material so bereitzustellen, daß es sowohl durch Licht als auch durch Vermischen zweier Komponenten zu härten ist.

Die erfindungsgemäßen Kompositmaterialien weisen bevorzugt einen Polymerisationsschrumpf < 2,0 Vol. %, insbesondere < 1,8 Vol. %, ganz besonders < 1,6 Vol. % (gemessen nach der Bonded-Disc-Methode - Dental Materials (2004) 20, 88 - 95) auf.

Erfndungsgemäße Kompositmaterialien zeigen als Dentalmaterialien eine Schrumpfkraft (gemessen nach der photoelastischen Methode; Dental Materials (2004) 20, 313-321) von < 4,0, insbesondere < 3,75, ganz besonders < 3,5 MPa (gemessen 24 h nach Polymerisation).

Bisherige klassische Dentalmaterialien weisen folgende Schrumpfkräfte auf (gemessen 24 h nach Polymerisation):

| | |
|---|---|
| Filtek^{®} Supreme (Fa. 3M Espe) | 4,23 MPa |
| Grandio^{®} (Fa. Voco) | 5,68 MPa |
| Venus^{®} ( Fa. Heraeus Kulzer) | 5,55 MPa |
| Tetric^{®} Ceram (Fa. Ivoclar Vivadent) | 4,35 MPa |

### Beispiel

Ein Kompositmaterial wird durch innige Vermischung folgender Komponenten hergestellt aus

| | | | |
|---|---|---|---|
| Füllstoffe: | Nicht-agglomerierte Nanopartikel | 6 | Gew. Teile |
| | Dentalglas 1 µm (silanisiert) | 24 | Gew. Teile |
| | Dentalglas 8 µm (silanisiert) | 53 | Gew. Teile |
| Monomere: | Bis-GMA (Bowen) | 11 | Gew. Teile |
| | UDMA | 4 | Gew. Teile |
| | TEDMA | 2 | Gew. Teile |
| Initiator(en): | Campherchinon | 0,1 | Gew. Teile |
| Summe | | 100,1 | Gew. Teile |

Nach 60 sec. Polymerisation unter Licht einer Trans Lux CL - Lampe (Lichtleistung ca. 200 - 400 mW/cm²) ergeben sich die folgenden Werte:
Polymerisationschrumpf (Bonded-Disc-Methode): 1,48 Vol. %

Nach 60 sec. Polymerisation unter Licht einer Trans Lux Energy Lampe (Lichtleistung ca. 800 mW/cm² und Einlagerung 1 h nach Polymerisation in dest. Wasser bei 37 °C) ergeben sich die folgenden Werte:
Schrumpfkraft (24 h): 3,74 MPa

Das Material ist mit diesen Werten den oben erwähnten klassischen Dentalmaterialien überlegen.

## Patentansprüche

1. Kompositmaterial mit einem Gesamtfüllstoffgehalt von 80 bis 95 Gew.% enthaltend
A) in der Füllstoffkomponente 0,5 bis 10 Gew % nicht-agglomerierte Nanofüller mit Partikelgrößen von 1 bis 50 nm,
B) in der Füllstoffkomponente mindestens 60 Gew % eines Füllstoffgemischs aus 50 bis 90 % grob- und 10 bis 50% feinteiligen Dentalgläsern, welche ein Größenverhältnis, bezogen auf die mittlere Partikelgröße (d₅₀-Wert), von feinteilig zu grobteilig von 1:4 bis 1:30 aufweisen,
C) als Monomerkomponente eine Monomermischung aus
i. 60 - 80 % Bis-GMA oder TCD-di-HEMA oder TCD-di-HEA,
ii. 10 bis 18 % UDMA,
iii.Rest TEDMA und/oder multifunktionelle Vernetzer,
D) bis 1 Gew. % Initiator(en).

2. Kompositmaterial nach Anspruch 1, enthaltend D) bis 1 Gew. % Photoinitiator(en) zur Aushärtung mit Licht.

3. Kompositmaterial nach Anspruch 1, aufgeteilt in zwei Komponenten, enthaltend D) Initiatoren für die Kalt- oder Heißhärtung zur Aushärtung durch Vermischen.

4. Kompositmaterial nach Anspruch 1, enthaltend E) zusätzlich Photoinitiatoren zur Aushärtung durch Vermischen in Kombination mit Lichthärtung.

5. Kompositmaterial nach Anspruch 1, bei dem das Größenverhältnis feinteilig zu grobteilig im Bereich von 1:4 bis 1:20 liegt.

6. Kompositmaterial nach Anspruch 1, bei dem das Größenverhältnis feinteilig zu grobteilig 1:5 bis 1: 10 beträgt.

7. Kompositmaterial nach einem der vorstehenden Ansprüche, enthaltend zusätzlich F) in der Füllstoffkomponente mindestens ein weiteres Dentalglas mit sich von den grob- und feinteiligen Dentalgläsern unterscheidender Partikelgröße.

8. Kompositmaterial nach Anspruch 1, bei dem die Komponente D zu bis 0,3 Gew.% vorliegt.

9. Kompositmaterial nach Anspruch 1, bei dem die Komponente D zu bis 0,1 Gew.% vorliegt.

10. Kompositmaterial nach Anspruch 1, bei dem die mittlere Partikelgröße der grobteiligen Dentalgläser 7 µm und der feinteiligen 1 µm beträgt.

11. Kompositmaterial nach Anspruch 1, bei dem die Nanofüller der Gruppe bestehend aus SiO₂, ZrO₂, TiO₂ , Al₂O₃ sowie Mischungen aus mindestens zwei dieser Stoffe angehören.

12. Kompositmaterial nach einem der vorstehenden Ansprüche, mit einem Polymerisationsschrumpf von < 2,0 Vol. %, gemessen nach der Bonded-Disc-Methode [Dental Materials 20, 88 - 95 (2004)].

13. Kompositmaterial nach Anspruch 12, mit einem Polymerisationsschrumpf von < 1,8 Vol. %.

14. Kompositmaterial nach Anspruch 12, mit einem Polymerisationsschrumpf von < 1,6 Vol. %.

15. Verwendung eines Kompositmaterials nach einem der vorstehenden Ansprüche als Dentalmaterial.

## Claims

1. Composite material having a total filler content of 80 to 95% by weight containing
A) 0.5 to 10% by weight of non-agglomerated nanofillers with particle sizes of 1 to 50 nm in the filler components,
B) at least 60% by weight of a filler mixture of 50 to 90% coarse particles and 10 to 50% fine particle dental glasses in the filler components and having a size ratio relative to the average particle size (d50 value) of fine to coarse particles of 1:4 to 1:30,
C) as monomer components, a monomer mixture consisting of
i. 60 - 80 % bis-GMA or TCD-di-HEMA or TCO-di-HEA,
ii. 10 to 18% UDMA,
iii. balance TEDMA and/or multifunctional cross-linkers,
D) up to 1 % by weight initiator(s).

2. Composite material according to claim 1 containing D) up to 1 % by weight photo initiator(s) for curing by light.

3. Composite material according to claim 1 divided into two components containing D) initiators for cold or hot curing for the purpose of curing by admixture.

4. Composite material according to claim 1 containing E) also photo initiators for curing by admixture in combination with light curing.

5. Composite material according to claim 1 wherein the size ratio of fine to coarse particles is in the region of 1:4 to 1:20.

6. Composite material according to claim 1, wherein the size ratio of fine to coarse particles is 1:5 to 1: 10

7. Composite material according to one of the foregoing claims additionally containing F) in the filler component at least one further dental glass having a different particle size than the coarse and fine particle size.

8. Composite material according to claim 1 wherein component D is present in a proportion up to 0.3 % by weight.

9. Composite material according to claim 1, wherein component D is present in a proportion up to 0.1 % by weight.

10. Composite material according to claim 1 wherein the average particle size of the coarse-particle dental glass is 7 µm and the average particle size of the fine-particle dental glass is 1 µm.

11. Composite material according to claim 1 wherein the nanofillers belong to the group consisting of SiO₂, ZrO₂, TiO₂ , Al₂O₃ and admixtures of at least two of these substances.

12. Composite material according to one of the above claims having a polymerisation shrinkage of < 2.0% by volume measured according to the bonded disk method [Dental Materials 20, 88-95 (2004)].

13. Composite material according to claim 12 having a polymerisation shrinkage of < 1.8% by volume.

14. Composite material according to claim 12, having a polymerisation shrinkage of < 1.6% by volume

15. The use of a composite material according to one of the above claims as a dental material

## Revendications

1. Matériau composite avec une teneur en charge totale de 80 à 95 % en poids contenant
A) 0,5 à 10 % en poids de nanocharges non agglomérées avec des tailles particulaires de 1 à 50 nm dans le composant de charge,
B) au moins 60 % en poids d'un mélange de charges constitué de 50 à 90 % de verres dentaires à particules grossières et 10 à 50 % de verres dentaires à particules fines dans le composant de charge qui présentent une proportion par rapport à la taille particulaire moyenne (valeur d₅₀) entre particules fines et particules grossières de ¼ à 1/30,
C) un mélange de monomères constitué de
i. 60 à 80 % de bis-GMA ou TCD-di-HEMA ou TCD-di-HEA,
ii. 10 à 18 % de UDMA,
iii. le reste de TEDMA et/ou d'agents de réticulation multifonctionnels en tant que composant monomère,
D) jusqu'à 1 % en poids d'initiateur(s).

2. Matériau composite selon la revendication 1, contenant D) jusqu'à 1 % en poids de photo-initiateur(s) en vue du durcissement à la lumière.

3. Matériau composite selon la revendication 1, divisé en deux composants, contenant D) des initiateurs pour le durcissement à froid ou à chaud en vue du durcissement par mélange.

4. Matériau composite selon la revendication 1, contenant E) en outre des photo-initiateurs en vue du durcissement par mélange en combinaison avec un durcissement à la lumière.

5. Matériau composite selon la revendication 1, dans lequel la proportion entre particules fines et particules grossières se situe dans la plage de ¼ à 1/20.

6. Matériau composite selon la revendication 1, dans lequel la proportion entre particules fines et particules grossières est de 1/5 à 1/10.

7. Matériau composite selon l'une quelconque des revendications précédentes, contenant en outre F) au moins un verre dentaire supplémentaire dans le composant de charge avec une taille particulaire variant entre les verres dentaires à particules grossières et fines.

8. Matériau composite selon la revendication 1, dans lequel le composant D est présent jusqu'à 0,3 % en poids.

9. Matériau composite selon la revendication 1, dans lequel le composant D est présent jusqu'à 0,1 % en poids.

10. Matériau composite selon la revendication 1, dans lequel la taille particulaire moyenne des verres dentaires à particules grossières est de 7 µm et celle des verres dentaires à particules fines est de 1 µm.

11. Matériau composite selon la revendication 1, dans lequel les nanocharges appartiennent au groupe constitué de SiO₂, ZrO₂, TiO₂, Al₂O₃ ainsi que des mélanges d'au moins deux de ces substances.

12. Matériau composite selon l'une quelconque des revendications précédentes, avec un retrait par polymérisation 2,0 % en volume, mesuré selon le procédé des disques collés [Dental Materials 20, 88 - 95 (2004)].

13. Matériau composite selon la revendication 12, avec un retrait par polymérisations < 1,8 % en volume.

14. Matériau composite selon la revendication 12, avec un retrait par polymérisation < 1,6 % en volume.

15. Utilisation d'un matériau composite selon l'une quelconque des revendications précédentes en tant que matériau dentaire.
